# EUROPEAN PATENT SPECIFICATION

(11) **EP 1 520 517 B1**
(45) Date of publication and mention of the grant of the patent: **14.12.2005**
(21) Application number: 03425638.8
(22) Date of filing: 30.09.2003
(51) Int. Cl.: A61B 8/08, G01S 7/52

(54) **Method for estimating tissue velocity vectors and tissue deformation from ultrasonic diagnostic imaging data**
Verfahren zur Bestimmung von Gewebedeformation und Gewebegeschwindigkeitsvektoren mittels Ultraschallbilddarstellung
Procédé pour estimer la déformation du tissu et les vecteurs de vitesse du tissu au moyen d'imagerie à ultrasons

(43) Date of publication of application: 06.04.2005
(73) Proprietor: Esaote S.p.A., 20123 Milano (IT); Amid SRL, 00191 Roma (IT)
(72) Inventor: Pedrizzetti, Gianni, 59100 Prato (IT); Tonti, Giovanni, 67039 Sulmona (IT)
(74) Representative: Karaghiosoff, Giorgio Alessandro

(56) References cited:
- WO-A-02/45587

## Description

The invention relates to a method for estimating tissue velocity vectors and tissue deformation, oriented strain, shear, from ultrasonic diagnostic imaging data, comprising the following steps:

Acquiring ultrasound imaging data from an object by transmitting ultrasound beams against the said object and receiving the corresponding reflected beams by the said object;

Defining a certain number of reference points in an ultrasonic image field corresponding to the ultrasonic image data obtained from evaluation of the ultrasonic reflected beams;

Determining the direction of motion and the velocity vector of the said reference points from the ultrasonic image data;

Evaluating the deformation state about the said reference points from the determined velocity data;

In many diagnostic evaluations of ultrasound images, the quantitative evaluation of the tissue kinematic properties (velocity and deformation) improves the ability to identify dysfunctions.

A field where this kind of analysis has a particular relevance is the field of echocardiographic diagnostic imaging. In this field, the assessment of the effective ventricular function requires the knowledge of numerous properties about the ventricular dynamics.

A recent technique for evaluating velocity is the so called Doppler Tissue Imaging, so called DTI. This technique allows to measure tissue velocity over all points in the ventricular wall. The measurement of velocity itself provides a direct information about the wall motion and helps to uncover abnormalities not immediately observable from the visualization in B-mode. The velocity contains information about either rigid body displacement and contraction/distension, the latter being immediately related to the myocardial activity. Post processing of the DTI velocity data allows the evaluation of additional quantities, namely strain-rate and strain, that are strictly related to the regional function. Segmental strain gives a direct evaluation of the degree of contractility of the myocardium during systole, as well as of its relaxation during ventricular filling.

Nevertheless DTI suffers from a few drawbacks consisting in limitations of the technique. The evaluation of velocity, and to a greater degree when strain rate and strain are evaluated , requires a higher frame rate with respect to be mode imaging because velocity is a more rapidly varying function than B-mode displacement. A Doppler signal requires additional processing with respect to the simple echo. Doppler tissue imaging suffers further of an intrinsic limitation due to the fact that only the component of velocity along a scanline can be measured. This limitation has several drawbacks. When tissue moves in a direction that is not aligned with the scanline, the Doppler velocity does not reflect the effective tissue kinematics. Only the component of strain and strain rate along the scanline can be evaluated, giving a reduced view of the local deformation state. This limits the application of DTI to the anatomic sites that can be imagined aligned along a scanline. In echocardiography this corresponds essentially to the interventricular septum and to the lateral walls in apical view.

A strain rate analysis method in ultrasonic diagnostic imaging applying the above mentioned DTI technique is disclosed in WO 02/45587. According to this document strain rate analysis is performed for ultrasonic images in which the spatial gradient of velocity is calculated in the direction of tissue motion. Strain rate is calculated for cardiac ultrasound images in the direction of motion which, for myocardial images , may be either in the plane of the myocardium or across the myocardium. Strain rate information is calculated for a sequence of images of a heart cycle and displayed for an automatically drawn border such as the endocardial border over the full heart cycle. Using DTI technique the method of document WO02/45587 suffers of the same drawbacks as the DTI technique. Furthermore WO02/45587 teaches how to carry out the automatic drawing of a border and the successive tracking of the said border during its motion. In any case such a method is affected, as all the other border detection methods that are based on arbitrary definitions of border, by a non complete reliability and thus by a rare practical use in clinic diagnosis, since the imaged structures are often not so easy to be determined.

From the fluid dynamics perspective, a velocity field estimation method is known called Particle Image Velocimetry. According to this method a sequence of grey scale images are taken on an illuminated slice of a fluid seeded with non buoyant micro particles, for measuring the velocity of the said micro-particles out form the sequence of images. The method is an optical flow method and, as such, it is based on the assumption of conservation of brightness. According to this assumption, an object (a patch of brightness) displaces without local changes from one image frame to the consecutive frame. PIV is actually well suited for fluid motion where relevant deformations are present and it has widely employed in measuring turbulent flow showing a good reliability in such extreme conditions when explicit subject is not clearly identifiable. For better understanding of PIV see Adrian RJ, Particle-Image technique for experimental fluid mechanics Annu. Rev. Fluid Mech. 1991, 23; 261; Melling A, Tracer particles and seeding for particle image velocimetry Meas. Sci. Technol. 1997, 8; 1406. Singh A. Optic Flow Computation: A unified Perspective. Piscataway, NJ; IEEE Comput. Soc. Press, 1992; Barrow JL, Fleet DJ, Beuchermin S. Performance of optical flow techniques, International Journal of Computer Vision 1994, 12;43-77; Hu H. Saga, T. Kobayashi, T.Taniguchi, N.Research on the vertical and turbulent structures in the lobed jet flow by using LIF and PIV. Meas.Sci. Technolo. 2000, 1; 698 and BrowneP, Ramuzat A, Saxena R, Yoganathan AP.

The invention aims to provide for a method for estimating tissue velocity vectors and deformation state, strain, shear, from ultrasonic diagnostic imaging data, which does not need to acquire ultrasonic image data in Doppler mode and which can obviate to the drawbacks of the velocity and strain evaluation carried out starting from the said Doppler mode ultrasonic data, still furnishing a reliable and precise evaluation of the velocity vector and of the deformation tensor.

Furthermore the invention aims to provide for an evaluation of velocity vectors and strain data, which allows to determine velocity components which are transversal to the scanline and all of the independent components of a two dimensional strain consisting in the longitudinal strain along two orthogonal axis, particularly along the tissue and across the thickness, and in the shear.

According to the present invention a method for estimating tissue velocity vectors and deformation from ultrasonic diagnostic imaging data, comprises the following steps:

Acquiring ultrasound imaging data from an object by transmitting ultrasound beams against the said object and receiving the corresponding reflected beams by the said object;

Defining a certain number of reference points in an ultrasonic image field corresponding to the ultrasonic image data obtained from evaluation of the ultrasonic reflected beams;

Determining the direction of motion and the velocity vector of the said reference points from the ultrasonic image data;

The ultrasound imaging data consist in a sequence of at least two image frames;

The imaging data are B-mode grey-scale echographic images;

The velocity of motion of each reference point between two successive B-mode image frames are determined by applying a so called optical flow method, like, for example, the particle image velocimetry technique abbreviated as PIV.

More particularly the particle image velocimetry applied in the present invention comprises the following steps:
a) Acquiring at least two consecutive B-mode and grey scale image frames along an ultrasound scanline;
b) Defining at least an identical point in the at least two consecutive image frames;
c) Defining a small region of NxN pixels where N=natural number which region is centered on the said point;
d) Carrying out a first displacement and velocity estimation cycle of the said point between the first and the second frame of the said at least two consecutive frames by calculating the cross-correlation between the two small regions in the two consecutive frames and determining the position where the local correlation of the two consecutive frames is a maximum;
e) Determining the displacement of the said maximum from the first frame to the second frame of the said two consecutive frames and defining the said displacement as the displacement of the point from the position of the point in the first frame to position of the said point in the second frame of the said two consecutive frames;
f) Calculating the velocity as the quotient of the displacement of the point from the position of the said point in the first of the two consecutive frames to the position of the said point in the second of the said two consecutive frames and the time distance between the said two consecutive frames.
g) Evaluating the deformation from time integration of the appropriate component of the velocity gradient, the latter evaluated by estimating the spatial derivatives from velocity in two or more points.

In a further improvement a sequence of more than two consecutive frames are acquired and the displacement of a certain defined point and the velocity is evaluated as described before for each couple of consecutive frames in the said sequence of consecutive frames.

According to still another improvement more than one point is defined and the said method steps as described before are applied to each one of the said points.

Considering a local region of the image frames acquired of NXN pixels for a certain point X centered in the said region in the first of the consecutive frames of a sequence of frames, the above disclosed first estimation cycle would lead to the determination of a sequence of displacements Δxᵢ for the said point X from a frame i to the following frame i+1.

The velocity of the point X in the displacement from the frame I to the following consecutive frame i+1 of the sequence of frames is than Δxᵢ/Δtᵢ which is the time interval between the two consecutive frames i and i+1.

If necessary the above mentioned method steps consisting in a first estimation cycle of the displacements of a certain defined point or of certain defined points and of their velocity along the sequence of consecutive frames can be followed by further cycles for improving the precision.

The said further cycles for improving the precision of the estimation comprising the following steps:
Repeating at least a second time or more than a second time the above disclosed method steps c) to f) by defining a new small region in each frame of the said sequence of consecutive frame as the square regions centered at the defined point displaced by the corresponding displacement of the point from one first frame to the consecutive frame in the said sequence of frames as calculated in the first or previous estimation cycle;
Carrying out the cross correlation between the said regions and determining as in the first estimation cycle a second displacement value;
Adding the said second displacement value for each couple of consecutive frames to the first displacement value calculated in the first cycle for the said couple of consecutive frames.

Considering the further example related on the point X for second frame of the two consecutive frames the local region of NxN pixels, for example of 64x64 pixels, is displaced form the first frame i to the consecutive frame i+1 by Δxᵢ.

According to a further improvement, the region in the first frame i is chosen centered at X-Δxᵢ/2 and the region in the following frame i+1 is chosen centered at X+Δxᵢ/2. This two regions of the two consecutive image frames are now approximately centered on the same moving point, or object and the cross correlation of the two regions gives a correction value to the displacement of point X evaluated according to the first estimation cycle which has to be added to the said displacement value according to the first estimation cycle.

Further iterations of the said cycle can be carried out till a certain minimum value of the correction of the displacement is reached.

According to a further improvement, after the last iteration the maximum of the local correlation between the position of the defined point in the two consecutive frames is further individuated by interpolating the computed values of cross-correlations in order to achieve a sub-pixel precision in the determination of the velocity.

According to still another feature of the method the small region in the image frames is chosen in such a way as the number N of pixels in each of the two spatial directions is great enough to contain any possible displacement of the chosen point in the first frame along the sequence of consecutive frames.

During the iteration process, according to another feature, the NxN small image region can be reduced in size at each iteration in order to improve resolution. For example employing n consecutive division by 2,the result is an estimation of velocity on a region 2⁻ⁿNx2⁻ⁿN, N and n may vary in different applications. Typically N=64 and n=2 gives acceptable precisions.

According to a further feature, a validation of data, for example a nonlinear median filter, is applied to the result to ensure a spatial and time coherence of results and eliminate the possibility of outliers points produced by image noise.

The method according to the invention has particular relevance for echocardiographic images. The method according to the invention is not limited by the scanline direction as the Doppler Tissue Imaging method and allows therefore to evaluate velocity also in a transversal direction to the scanline direction.

This is a considerable advantage, since in applying the method according to the present invention the relative position of the probe and of the imaged object is not critical as for evaluation of velocities according to the traditional Doppler Tissue Imaging.

Comparisons of the results obtained by the method according to the present invention with the results obtained by velocity estimation by means of the DTI technique are in very good agreement.

The method according to the present invention allows to gain knowledge of all the components of velocity vector along an arbitrary direction. This is very relevant for strain computation from the velocity data and allows evaluation of contraction/relaxation of tissues (longitudinal strain) along a arbitrary oriented line.

The evaluation of the two components of a two dimensional velocity vector allows observations of tissue motion and of dynamic properties that cannot be analyzed otherwise, like the simultaneous record of transversal and longitudinal velocity components. The analysis of the complete tissue dynamics from an y scan plane projection, including short axis is also made possible. Also the evaluation of strain and of strain rate oriented along an arbitrary direction and, if required, of the tissue shear is made possible.

The method can be applied without conceptual change to sequence of three dimensional imaging data, or volumetric data set. In this case the image area NxN is substituted by a volume NxNxN. The correlation between two consecutive three-dimensional fields are identically defined mathematically, the result of the said procedure is a displacement three-dimensional vector.

Application to three dimensional imaging allows the evaluation of the three dimensional velocity vector in one or more points, and the six components of deformation including elongation along any direction in space, and shear about any axis.

## Claims

1. A method for estimating tissue velocity vectors and oriented strain from ultrasonic diagnostic imaging data, comprises the following steps:
Acquiring ultrasound imaging data from an object by transmitting ultrasound beams against the said object and receiving the corresponding reflected beams by the said object;
Defining at least one or a certain number of reference points in an ultrasonic image field corresponding to the ultrasonic image data obtained from evaluation of the ultrasonic reflected beams;
Determining the direction of motion and the velocity vector of the said reference points from the ultrasonic image data;
**Characterised in that**
The ultrasound imaging data consist in a sequence of at least two image frames, said images being two dimensional images or three dimensional data;
The imaging data are B-mode grey-scale echographic images;
The velocity of motion of each reference point between two successive B-mode image frames are determined by applying a so called particle image velocimetry technique abbreviated as PIV.
Any component of the strain is then obtained from time integration of strainrate that is evaluated from the gradient of velocity estimated from the velocity data in two or more points.

2. A method according to claim 1 in which the following steps are provided:
a) Acquiring at least two consecutive B-mode and grey scale image frames along an ultrasound scanline;
b) Defining at least an identical point in the at least two consecutive image frames;
c) Defining a small region of NxN pixels where N=natural number which region is centered on the said point;
d) Carrying out a first displacement and velocity estimation cycle of the said point between the first and the second frame of the said at least two consecutive frames by calculating the cross-correlation between the two small regions in the two consecutive frames and determining the position where the local correlation of the two consecutive frames is a maximum;
e) Determining the displacement of the said maximum from the first frame to the second frame of the said two consecutive frames and defining the said displacement as the displacement of the point from the position of the point in the first frame to position of the said point in the second frame of the said two consecutive frames;
f) Calculating the velocity as the quotient of the displacement of the point from the position of the said point in the first of the two consecutive frames to the position of the said point in the second of the said two consecutive frames and the time distance between the said two consecutive frames.

3. A method according to claim 2 **characterised in that** a sequence of more than two consecutive frames are acquired and the displacement of a certain defined point and the velocity is evaluated as described before for each couple of consecutive frames in the said sequence of consecutive frames.

4. A method according to claims 2 or 3 **characterised in that** more than one point is defined and the said method steps as described before are applied to each one of the said points.

5. A method according to claims 4 with the following step (g) integrating those described in claim 2:
g) Evaluating the deformation, strain, shear, from time integration of the appropriate component of the velocity gradient, the latter evaluated by estimating the spatial derivatives from velocity in two or more points.

6. A method according to one or more of the preceding claims **characterised in that** the steps c) to f) consisting in a first estimation cycle of the displacements of a certain defined point or of certain defined points and of their velocity along the sequence of consecutive frames can be followed by further cycles for improving the precision, the said further cycles for improving the precision of the estimation comprising the following steps:
Repeating at least a second time or more than a second time the above disclosed method steps c) to f) by defining a new small region in each frame of the said sequence of consecutive frame as the square regions centered at the defined point displaced by the corresponding displacement of the point from one first frame to the consecutive frame in the said sequence of frames as calculated in the first or previous estimation cycle;
Carrying out the cross correlation between the said regions and determining as in the first estimation cycle a second displacement value;
Adding the said second displacement value for each couple of consecutive frames to the first displacement value calculated in the first cycle for the said couple of consecutive frames.

7. A Method according to claim 6, **characterised in that** further iterations of the said cycle are carried out till a certain minimum value of the correction of the displacement is reached.

8. A method according to claim 6 or 7 **characterised in that** after the last iteration the maximum of the local correlation between the position of the defined point in the two consecutive frames is further individuated by interpolating the computed values of cross-correlations in order to achieve a sub-pixel precision in the determination of the velocity.

9. A method according to one or more of the preceding claims 6 to 8, **characterised in that** the small region in the image frames is chosen in such a way as the number N of pixels in each of the spatial directions is great enough to contain any possible displacement of the chosen point in the first frame along the sequence of consecutive frames.

10. A method according to one or more of the preceding claims 6 to 9, **characterised in that** during the iteration process of the estimation cycle, the small image region can be reduced in size at each iteration in order to improve resolution.

11. A method according to one or more of the preceding claims **characterised in that** a validation method is applied to the result to eliminate the possibility of outliers points.

## Patentansprüche

1. Verfahren zur Schätzung der Geschwindigkeitsvektoren von Gewebe und der richtungsbezogenen Dehnung mittels Daten der Ultraschalldiagnostik-Bildgebung, das folgende Verfahrensschritte umfaßt:
Erfassung der Ultraschallbildgebungsdaten eines Objekts durch das Senden von Ultraschallstrahlen auf das besagte Objekt und den Empfang der entsprechenden von dem besagten Objekt reflektierten Strahlen;
Bestimmung von mindestens einem oder einer bestimmten Anzahl von Referenzpunkten in einem Ultraschallbildfeld, das den durch Auswertung der reflektierten Ultraschallstrahlen erhaltenen Ultraschallbildgebungsdaten entspricht;
Bestimmung der Bewegungsrichtung und des Geschwindigkeitsvektors der besagten Referenzpunkte aus den Daten der Ultraschallbildgebung;
**dadurch gekennzeichnet, daß**
die Daten der Ultraschallbildgebung eine Abfolge mindestens zweier Bilder ergeben, wobei die besagten Bilder zweidimensionale Bilder oder dreidimensionale Daten sind;
die Bilddaten graustufige Echographiebilder im B-Mode sind;
die Bewegungsgeschwindigkeit eines jeden Referenzpunkts zwischen zwei aufeinander folgenden B-Mode-Bildern durch Anwendung des sogenannten PIV-Verfahrens (PIV = Particle Image Velocimetry) bestimmt wird.
Die Komponenten der Dehnung erhält man dann aus der Zeitintegration der Dehnungsrate, die aus dem durch Schätzung der Geschwindigkeitsdaten an zwei oder mehr Punkten ermittelten Geschwindigkeitsgradienten ermittelt wird.

2. Verfahren nach Anspruch 1 mit folgenden Verfahrensschritten:
a) Erhalt von mindestens zwei aufeinander folgenden B-Mode- und Graustufenbildern an einer Ultraschallscanlinie;
b) Bestimmung von mindestens einem identischen Punkt in den mindestens zwei aufeinander folgenden Bildern;
c) Bestimmung eines kleinen Bereichs von NxN Pixel, wobei N eine natürliche Zahl ist und die Mitte dieses Bereichs im besagten Punkt liegt;
d) Durchführung eines ersten Ablaufs zur Schätzung der Verschiebung und Geschwindigkeit des besagten Punkts zwischen dem ersten und zweiten Bild der besagten mindestens zwei aufeinander folgenden Bilder, indem die Kreuzkorrelation zwischen den zwei kleinen Bereichen in den zwei aufeinander folgenden Bildern errechnet wird und die Position bestimmt wird, bei welcher die lokale Korrelation der zwei aufeinander folgenden Bilder ihren Maximalwert erreicht;
e) Bestimmung der Verschiebung des besagten Maximalwerts vom ersten Bild zum zweiten Bild der besagten beiden aufeinander folgenden Bilder und Definition der besagten Verschiebung als die Verschiebung des Punkts von der Punktposition im ersten Bild zur Position des besagten Punkts im zweiten Bild der besagten beiden aufeinander folgenden Bilder;
f) Berechnung der Geschwindigkeit als Quotient der Verschiebung des Punkts von der Position des besagten Punkts im ersten der beiden aufeinander folgenden Bilder zur Position des besagten Punkts im zweiten der besagten beiden aufeinander folgenden Bilder und des Zeitabstands zwischen den besagten beiden aufeinander folgenden Bildern.

3. Verfahren nach Anspruch 2,
**dadurch gekennzeichnet, daß**
eine Abfolge von mehr als zwei aufeinander folgenden Bildern erfaßt und die Verschiebung eines bestimmten Punkts sowie die Geschwindigkeit für jedes Paar aufeinander folgender Bilder in der besagten Abfolge aufeinander folgender Bilder, wie zuvor beschrieben, ermittelt wird.

4. Verfahren nach Anspruch 2 oder 3,
**dadurch gekennzeichnet, daß**
mehr als ein Punkt bestimmt wird und die besagten Verfahrensschritte, wie zuvor beschrieben, auf jeden einzelnen der besagten Punkte angewendet werden.

5. Verfahren nach Anspruch 4 mit dem nachstehenden Schritt g) zur Einbindung der in Anspruch 2 beschriebenen Schritte:
g) Ermittlung von Deformation, Dehnung und Scherung aus der Zeitintegration der entsprechenden Komponente des Geschwindigkeitsgradienten, wobei letzterer durch Schätzung der räumlichen Ableitungen von der Geschwindigkeit an zwei oder mehr Punkten ermittelt wird.

6. Verfahren nach einem oder mehreren der vorstehenden Ansprüche,
**dadurch gekennzeichnet, daß**
nach den Schritten c) bis f), die einen ersten Ablauf zur Schätzung der Verschiebungen eines bestimmten Punkts oder bestimmter Punkte und deren Geschwindigkeit in der Abfolge der aufeinander folgenden Bilder darstellen, weitere Abläufe zur Verbesserung der Präzision erfolgen können, wobei die besagten weiteren Abläufe zur Verbesserung der Präzision der Schätzung folgende Schritte beinhalten:
Wiederholung der vorstehend genannten Verfahrensschritte c) bis f) mindestens ein zweites Mal oder mehr als nur ein zweites Mal, indem erneut ein kleiner Bereich in jedem Bild der besagten Abfolge der aufeinander folgenden Bilder jeweils als quadratförmiger Bereich festgelegt wird, dessen Mitte in dem definierten Punkt liegt und der um den entsprechenden, bereits beim ersten oder vorhergehenden Schätzungsablauf berechneten Weg vom Punkt im ersten Bild bis zum darauf folgenden Bild der besagten Bildfolge verschoben ist;
Durchführung der Kreuzkorrelation zwischen den besagten Bereichen und Bestimmung eines zweiten Werts der Verschiebung wie beim ersten Schätzungsablauf;
Hinzuaddieren des besagten zweiten Werts der Verschiebung für jedes Paar aufeinander folgender Bilder zum ersten Wert der Verschiebung, der beim ersten Ablauf für das besagte Paar aufeinander folgender Bilder berechnet wurde.

7. Verfahren nach Anspruch 6,
**dadurch gekennzeichnet, daß**
weitere Iterationen des besagten Ablaufs durchgeführt werden, bis ein bestimmter Minimalwert der Verschiebungskorrektur erreicht ist.

8. Verfahren nach Anspruch 6 oder 7,
**dadurch gekennzeichnet, daß**
der Maximalwert der lokalen Korrelation zwischen der Position des definierten Punkts in den zwei aufeinander folgenden Bildern nach der letzten Iteration noch näher bestimmt wird, indem die berechneten Werte der Kreuzkorrelationen interpoliert werden, um bei der Geschwindigkeitsermittlung eine Präzision im Subpixelbereich zu erlangen.

9. Verfahren nach einem oder mehreren der vorstehenden Ansprüche 6 bis 8,
**dadurch gekennzeichnet, daß**
der kleine Bereich in den Bildern so ausgewählt wird, daß die Anzahl N der Pixel in jeder räumlichen Richtung ausreichend groß ist, damit alle möglichen Verschiebungen des im ersten Bild ausgewählten Punkts in der Abfolge der nachfolgenden Bilder enthalten sind.

10. Verfahren nach einem oder mehreren der vorstehenden Ansprüche 6 bis 9,
**dadurch gekennzeichnet, daß**
der kleine Bildbereich während des Iterationsprozesses zur Durchführung der Schätzung bei jeder Iterationsstufe weiter verkleinert werden kann, um so die Auflösung zu erhöhen.

11. Verfahren nach einem oder mehreren der vorstehenden Ansprüche,
**dadurch gekennzeichnet, daß**
auf das Ergebnis eine Validierungsmethode angewendet wird, um mögliche Ausreißerpunkte auszuschließen.

## Revendications

1. Procédé d'estimation des vecteurs vitesse de tissu et du champ de contrainte à partir de données d'imagerie de diagnostic par ultrasons, comprenant les étapes suivantes :
Acquisition des données d'imagerie par ultrasons à partir d'un objet, en envoyant des faisceaux d'ultrasons sur ledit objet et en recevant les faisceaux réfléchis par ledit objet correspondants ;
Définition d'au moins un ou d'un certain nombre de points de référence dans un champ image ultrasonique correspondant aux données d'image ultrasonique obtenues à partir de l'évaluation des faisceaux ultrasoniques réfléchis ;
Détermination de la direction du mouvement et du vecteur vitesse desdits points de référence à partir des données d'image ultrasonique ;
**caractérisé en ce que**
les données d'imagerie par ultrasons consistent en une séquence de deux images tramées au moins, lesdites images étant des images bidimensionnelles ou des données tridimensionnelles ;
les données d'imagerie sont des images d'échographie en échelle de gris en mode B ;
la vitesse du mouvement de chaque point de référence entre deux cadres images tramées en mode B successives est déterminée en appliquant ce qu'il est convenu d'appeler une technique de vélocimétrie par images de particules, désignée de manière abrégée par l'acronyme PIV ;
n'importe quelle composante de la contrainte est alors obtenue à partir de l'intégration par rapport au temps de la vitesse de déformation qui est évaluée à partir du gradient de vitesse estimé à partir des données de vitesse en deux points ou plus.

2. Procédé selon la revendication 1, dans lequel les étapes suivantes sont prévues :
a) Acquisition d'au moins deux images tramées consécutives en mode B et en échelle de gris le long d'une ligne de balayage des ultrasons ;
b) Définition d'au moins un point identique dans les deux au moins images tramées consécutives ;
c) Définition d'une petite région de N × N pixels où N est un entier naturel, laquelle région est centrée sur ledit point ;
d) Réalisation d'un premier cycle d'évaluation du déplacement et de la vitesse dudit point entre les première et deuxième images desdites deux au moins images consécutives en calculant la corrélation croisée entre les deux petites régions dans les deux images consécutives et en déterminant la position où la corrélation locale des deux images consécutives est un maximum ;
e) Détermination du déplacement dudit maximum de la première image vers la deuxième image desdites deux images consécutives et détermination dudit déplacement comme étant le déplacement du point à partir de la position du point dans la première image vers la position dudit point dans la deuxième image desdites deux images consécutives ;
f) Calcul de la vitesse comme étant le quotient du déplacement du point à partir de la position dudit point dans la première des deux images consécutives vers la position dudit point dans la deuxième desdites deux images consécutives, sur l'écart temporel entre lesdites deux images consécutives.

3. Procédé selon la revendication 2, **caractérisé en ce qu'**une séquence de plus de deux images consécutives est acquise et **en ce que** le déplacement d'un certain point déterminé et la vitesse sont évalués comme cela a été décrit précédemment pour chaque couple d'images consécutives dans ladite séquence d'images consécutives.

4. Procédé selon les revendications 2 ou 3, **caractérisé en ce que** plus d'un point sont déterminés et **en ce que** lesdites étapes du procédé, telles qu'elles ont été décrites précédemment, sont appliquées à chacun desdits points.

5. Procédé selon la revendication 4, avec l'étape suivante (g) qui intègre celles décrites dans la revendication 2 :
g) Evaluation de la déformation, de la contrainte, du cisaillement, à partir de l'intégration par rapport au temps de la composante appropriée du gradient de vitesse, ce dernier étant évalué en estimant les dérivées spatiales à partir de la vitesse en deux points ou plus.

6. Procédé selon l'une ou plusieurs des revendications précédentes, **caractérisé en ce que** les étapes c) à f), consistant en un premier cycle d'estimation des déplacements d'un certain point déterminé ou de certains points déterminés et de leur vitesse tout au long de la séquence d'images consécutives, peuvent être suivies d'autres cycles pour améliorer la précision, lesdits autres cycles pour améliorer la précision de l'estimation comprenant les étapes suivantes :
Répétition au moins une deuxième fois, ou plus d'une deuxième fois, des étapes c) à f) du procédé décrites ci-dessus, en définissant une nouvelle petite région dans chaque image de ladite séquence d'images consécutives comme les régions carrées centrées sur le point déterminé déplacé du déplacement correspondant du point de la première image à l'image consécutive dans ladite séquence d'images, tel que calculé dans le premier cycle d'estimation ou dans le cycle d'estimation précédent ;
Réalisation de la corrélation croisée entre lesdites régions et détermination, de même que dans le premier cycle d'estimation, d'une deuxième valeur de déplacement ;
Addition de ladite deuxième valeur de déplacement pour chaque couple d'images consécutives à la première valeur de déplacement calculée dans le premier cycle pour lesdits couples d'images consécutives.

7. Procédé selon la revendication 6, **caractérisé en ce que** d'autres itérations dudit cycle sont réalisées jusqu'à ce qu'une certaine valeur minimum de la correction du déplacement soit atteinte.

8. Procédé selon la revendication 6 ou 7, **caractérisé en ce qu'**après la dernière itération, le maximum de la corrélation locale entre la position du point déterminé dans les deux images consécutives est différencié de plus en interpolant les valeurs calculées des corrélations croisées afin d'atteindre une précision inférieure au pixel dans la détermination de la vitesse.

9. Procédé selon l'une ou plusieurs des revendications précédentes 6 à 8, **caractérisé en ce que** la petite région dans les images tramées est choisie de telle manière que le nombre N de pixels dans chacune des directions spatiales est assez grand pour contenir n'importe quel déplacement possible du point choisi dans la première image tout au long de la séquence d'images consécutives.

10. Procédé selon l'une ou plusieurs des revendications précédentes 6 à 9, **caractérisé en ce qu'**au cours du processus d'itération du cycle d'estimation, la taille de la petite région d'image peut être réduite à chaque itération afin d'améliorer la résolution.

11. Procédé selon l'une ou plusieurs des revendications précédentes, **caractérisé en ce qu'**un procédé de validation est appliqué au résultat pour éliminer la possibilité de points aberrants.
